# FASCICULE DE BREVET EUROPEEN

(11) **EP 2 577 317 B1**
(45) Date de publication et mention de la délivrance du brevet: **03.12.2014**
(21) Numéro de dépôt: 11728343.2
(22) Date de dépôt: 01.06.2011
(51) Int. Cl.: G01N 33/68, G01N 33/558

(54) **PROCÉDÉ DE DÉTECTION DE RUPTURE DE MEMBRANES FOETALES**
VERFAHREN ZUR ERKENNUNG VON BLASENSPRUNG
METHOD OF DETECTING RUPTURE OF FETAL MEMBRANES

(30) Priorité: 03.06.2010 FR 1054374
(43) Date de publication de la demande: 10.04.2013
(73) Titulaire: Biosynex (société À Responsabilité Limitée), 67201 Eckbolsheim (FR)
(72) Inventeur: PAPER, Thierry, F-67000 Strasbourg (FR); RAKOTONDRAMANGA, Françoise, F-67590 Schweighouse sur Moder (FR)
(74) Mandataire: Novagraaf Technologies
(86) Numéro de dépôt international: PCT/FR2011/051254
(87) Numéro de publication internationale: WO 2011/151597

(56) Documents cités:
- EP-A1- 0 565 541
- US-A1- 2006 240 495
- LINKOV F ET AL: "Early detection of head and neck cancer: Development of a novel screening tool using multiplexed immunobead-based biomarker profiling", CANCER EPIDEMIOLOGY BIOMARKERS AND PREVENTION, vol. 16, no. 1, janvier 2007 (2007-01), pages 102-107, XP002595784, ISSN: 1055-9965
- RUTANEN E-M ET AL: "EVALUATION OF A RAPID STRIP TEST FOR INSULIN-LIKE GROWTH FACTOR BINDING PROTEIN-1 IN THE DIAGNOSIS OF RUPTURED FETAL MEMBRANES", CLINICA CHIMICA ACTA, ELSEVIER BV, AMSTERDAM, NL LNKD- DOI:10.1016/0009-8981(96)80001-E, vol. 253, no. 1/02, 1 janvier 1996 (1996-01-01), pages 91-101, XP000874594, ISSN: 0009-8981 cité dans la demande
- VAN CORTENBOSCH B ET AL: "Depistage de l'embolie amniotique : vers un test diagnostique ?", ANNALES DE BIOLOGIE CLINIQUE, vol. 65, no. 2, mars 2007 (2007-03), pages 153-160, XP002595785, ISSN: 0003-3898

## Description

### Domaine technique

La présente invention se rapporte à un procédé de détection de rupture des membranes foetales et à l'utilisation d'un dispositif de mise en oeuvre du dit procédé.

La présente invention trouve, notamment une application dans le domaine médical, le domaine diagnostique, en particulier dans le domaine obstétrique.

Dans la description ci-dessous, les références entre crochets ([ ]) renvoient à la liste des références présentée à la fin du texte.

### Etat de la technique

Durant la grossesse, les femmes peuvent être soumises à différents facteurs et/ou événements susceptibles de mettre en danger la grossesse. La rupture des membranes foetales fait partie de ces événements, en particulier lorsque la rupture des membranes est prématurée.

L'origine de cette rupture est variable. Il peut s'agir, par exemple, d'un traumatisme direct ou indirect comme un coup porté au niveau abdominal, accident de circulation, rapports sexuels, etc. Elle peut être due également à une fragilité membranaire endogène (déficience en collagène, malnutrition, tabagisme), une grossesse gémellaire, un hydramnios, une complication liée à une amnioscopie, à la pose d'un laminaire au cours d'une interruption volontaire de grossesse (IVG). Elle peut être également d'origine infectieuse et due à une Chorioamniotite, une vulvo-vaginite ou une endométrite. Enfin, dans 60% des cas la cause de la rupture est inconnue.

La rupture des membranes foetales entraîne un écoulement du liquide amniotique et représente une « porte ouverte » à tous les agents pathologiques : bactéries, virus etc. Ces agents peuvent infecter, par exemple le contenu utérin, le foetus.

Par ailleurs, la perte de liquide amniotique entraîne une diminution de l'espace disponible pour le développement du foetus. Cette diminution peut, lorsque la rupture a lieu avant le délai de 24 semaines d'aménorrhées, mettre en péril le pronostic vital du foetus entraînant une fausse couche et/ou conduisant à la naissance d'un bébé non viable. Après ce délai de 24 semaines, le pronostic vital de l'enfant est toujours mis en cause aboutissant à un accouchement prématuré et/ou une infection du foetus pouvant conduire au déclenchement volontaire de l'accouchement. Par ailleurs, la diminution du volume du liquide amniotique peut provoquer des problèmes de malformation, atrophie des membres, anomalie de développement de différentes parties du corps.

Le diagnostic de la rupture des membranes foetales est essentiellement clinique. Il s'agit d'un examen au spéculum par lequel est visualisé un écoulement de liquide amniotique. Ce test diagnostique est un test peu sensible, peu adapté au diagnostic des microruptures et qui ne permet pas de détecter rapidement et facilement la rupture des membranes et qui est surtout lié à la capacité du praticien de pouvoir identifier/visualiser l'écoulement du liquide.

Il existe également d'autres tests, par exemple le test à la nitrazine ou le test à la diamine oxydase permettant de détecter une rupture des membranes foetales.

Cependant, ces tests ne sont pas fiables et peuvent entraîner l'obtention de résultats faux négatifs (les patientes pour lesquelles le résultat est un faux négatif présentent donc une grossesse à risque et ne sont pas pris en charge et/ou traitées) et faux positifs (les patientes pour lesquelles le résultat est faux positif ne présentent pas de grossesse à risque et peuvent être prise en charge de la même façon qu'une femme avec rupture avec notamment le risque d'un déclenchement de l'accouchement inutile). Le test à la Nitrazine, indicateur de pH, est soumis à de nombreuses interférences notamment celles du sang, du sperme, d'antiseptiques ou d'infections vaginales. Le test à la diamine oxydase fait appel à des techniques de mise en évidence basées sur la radioimmunologie et donc peu pratiques. Sa sensibilité varie de 84 à 91%. Sa spécificité est limitée par l'interférence du sang.

Des tests de diagnostic basés sur la détection de la protéine 1 de liaison au facteur de croissance insulinomimétique (Insulin-like growth factor-binding protein 1 : IGFBP1) par des anticorps spécifiques dans un prélèvement vaginal ou cervical ont été également développés et commercialisés. Cette protéine est présente dans le liquide amniotique à une concentration 100 à 1000 fois supérieure à celle du sérum maternel (Rutanen et al. Radioimmunoassy of placenta protein 12 : levels in amniotic fluid, cord blood and sérum of healthy adults, pregnant women and patients with trophoblastic disease. Am. J. Obstet. Gynecol. 1982 [1]). Des exemples de tests de l'art antérieur utilisant ce marqueur sont, par exemple des tests immunologiques décrits dans le brevet européen EP 0 565 541. Cependant, ces tests ne sont pas fiables car ils peuvent fournir des résultats faux négatifs qui sont dus par exemple, à une concentration faible d'IGFBP1 dans le liquide amniotique, à un effet crochet, à une dégradation de la protéine dans le vagin et/ou *in-vitro,* ou à un effet matrice. Ils peuvent aussi générer des résultats faussement positifs qui sont dus par exemple, à la présence résiduelle d'IGFBP1 provenant d'autres milieux biologiques que le liquide amniotique, notamment le sang ou les cellules déciduales du col lorsque celui-ci est mature.

Des tests de diagnostic basés sur la détection de l'alpha foeto protéine (AFP) par des anticorps spécifiques dans un prélèvement vaginal ou cervical ont été également décrits. Un exemple de test de l'art antérieur utilisant ce marqueur est décrit dans l'article de Rochelson (Rochelson et al. Rapid assay: possible application in the diagnosis of premature rupture of the membranes. Obstet Gynecol 1983;62:414-418 [2])).

Cependant, ce test n'est pas fiable car il peut fournir des résultats faux négatifs et faux positifs (sensibilité 93% et spécificité 94% chez des femmes à moins de 39 semaines d'aménorrhées).

Un autre exemple de test de l'art antérieur a été décrit par Kishida (Kishida et al. Diagnosis of prématuré rupture of the membranes in preterm patients, using an improved AFP kit : comparison with ROM-Check and/or nitrazine test. European Journal of Obstetrics and Gynecology and Reproductive Biology 69 (1996) 77-82) [12]. Sur une population de patientes dont le terme est compris entre 16 et 36 semaines, la sensibilité et la spécificité sur prélèvement vaginal étaient respectivement de 96,9% et 92,9%. Sur un prélèvement cervical, la sensibilité et la spécificité étaient respectivement de 100% et 85,7%. Ainsi et comme d'autre test de l'état de la technique, ce test n'est pas fiable car il fournit des résultats faux négatifs et faux positifs.

D'autres marqueurs de ruptures moins performants ont également été décrits comme l'hormone chorio gonadotrophine humaine (hCG) (Young-Han Kim et al. Vaginal fluid b-human chorionic gonadotropin level in the diagnosis of prématuré rupture of membranes. Acta Obstet Gynecol Scand 2005: 84: 802-805 [3]) ou la prolactine (PRL) (Koninckx PR et al. Prolactin concentration in vaginal fluid: a new method for diagnosing ruptured membranes. Br J Obstet Gynaecol 1981;88:607-610 [4]).

US 2006/0240495 décrit la détection dans un échantillon vaginal de plusieurs marqueurs choisis parmi, entre autres, l'AFP, la fibronectine foetale, l'IGFBP-1, la prolactine et l'hormone lactogène placentaire humaine pour indiquer l'état de l'environnement intra-amniotique (rupture de membranes foetales, infection ou inflammation intra-amniotique, maturation pulmonaire foetale). La combinaison de l'AFP et de l'IGFBP-1 pour détecter une rupture des membranes foetales n'est pas explicitement divulguée.

Il existe donc un réel besoin de trouver un procédé de détection de rupture des membranes foetales palliant ces défauts, inconvénients et obstacles de l'art antérieur, en particulier d'un procédé avec une meilleure sensibilité et/ou une meilleure spécificité permettant de réduire les coûts et d'améliorer la détection des ruptures des membranes foetales.

### Description de l'invention

La présente invention a précisément pour but de répondre à ces besoins et inconvénients de l'art antérieur en fournissant un procédé de détection *in-vitro* de rupture des membranes foetales.

En particulier, la présente invention a pour objet un procédé de détection *in-vitro* d'une rupture des membranes foetales comprenant une étape de recherche simultanée, dans un prélèvement vaginal ou cervical, de l'alpha foeto protéine (AFP) et de la protéine 1 de liaison au facteur de croissance Insulinomimétique (IGFBP-1) , procédé dans lequel une rupture de membrane foetale est détectée lorsque l'IGFBP-1 seule ou en combinaison avec l'AFP est détectée, l'absence de rupture est détectée lorsque ni l'IGFBP-1 ni l'AFP n'est détecté, et lorsque seule l'AFP est détectée, le procédé comprend éventuellement en outre une étape de détection de l'IGFBP-1 seule.

Selon l'invention, la détection de l'IGFBP1 peut être remplacée par la détection de toute autre protéine ayant été décrite comme étant identique à l'IGFBP-1, par exemple l'alpha 1 microglobuline placentaire (placental alpha microglobuline 1 (PAMG-1)), la protéine placentaire 12 (« Placental Protein 12 »), l'alpha 1 globuline endométriale associée à la grossesse (« Pregnancy associated endometrial alpha1 globulin » (alpha1 PEG)), la protéine 14 endométriale (« Endometrial Protein 14 » (EP14)) (Bell C. Secretory endometrial and decidual proteins : studies and clinical significance of a maternally derived group of pregnancy associated sérum proteins. Human Reproduction. Vol. 1-3 129-143. 1986 [13] ; Nazimova et al. Blood Sérum Content of PAMG-1 Protein Binding Insulin like Growth Factor 1 (Somatomedin C) in Patients with Diabetes Mellitus. Bullet. Experim. Biol. and Med. 166/9 September 1993 [14]).

Les inventeurs sont en effet les premiers à avoir découvert, notamment, que la détection des deux marqueurs est complémentaire et permet d'atteindre un taux de sensibilité plus élevé. En particulier, les inventeurs sont les premiers à avoir découvert que la détection simultanée des deux marqueurs que sont l'alpha foeto protéine (AFP) et la protéine 1 de liaison au facteur de croissance insulinomimétique (Insulin-like growth factor-binding protein 1) (IGFBP1) permet avantageusement d'augmenter la spécificité et la sensibilité de la détection de la rupture.

Selon l'invention par prélèvement vaginal ou cervical on entend, par exemple un prélèvement effectué au niveau de la paroi du vagin d'une femme enceinte, au niveau du fornix postérieur du vagin, au niveau du col des sécrétions vaginales ou cervico-vaginales,

Selon l'invention, le prélèvement peut être obtenu par tout procédé connu de l'homme du métier. Le prélèvement peut être obtenu, par exemple par pipetage à l'aide d'une pipette ou par frottement des parois de la cavité vaginale, par exemple avec un écouvillon stérile dont le bouton peut être du coton ou une fibre synthétique. Il peut s'agir par exemple de polyester.

Selon l'invention la recherche de l'AFP et de l'IGFBP-1 peut être effectuée, par exemple au moyen d'anticorps. La détection de chacun de ces marqueurs peut être effectuée par exemple, au moyen d'anticorps monoclonaux, d'anticorps polyclonaux, et/ou de tout anticorps disponible dans le commerce capable de se fixer spécifiquement sur l'AFP ou sur l'IGFBP-1.

Selon l'invention, les anticorps dirigés contre l'AFP (anticorps anti AFP) peuvent être, par exemple des anticorps anti AFP décrits dans la demande internationale WO 2004013180, les anticorps commercialisés par la société Anticorps en Ligne (numéro de catalogue ABIN93624), les anticorps commercialisés par la société Arista Biologicals (clone 1, numéro de catalogue ABAFP-0401 ; clone 2, numéro de catalogue ABAFP-0402 ; clone 3, numéro de catalogue ABAFP-0403 ; clone 4, numéro de catalogue ABAFP-0404), les anticorps commercialisés par la société Hytest (clone 5H7, clone 4A3 et clone XA2, numéro de catalogue 4F16 ; anticorps polyclonal, numéro de catalogue ABAFP-0501)

Selon l'invention, les anticorps dirigés contre l'AFP peuvent être marqués ou non. Le marquage est exposé ci-dessous. Les anticorps anti AFP utilisés en capture et ceux marqués doivent être dirigés contre des épitopes différents afin que le système sandwich puisse fonctionner.

Selon l'invention, la concentration de l'anticorps anti AFP peut être par exemple de 0,1 à 100 µg/mL ou de 0,1 à 10 mg/ml.

Selon l'invention, le pH de solution comprenant les anticorps anti AFP peut être de 5 à 10, de préférence de 6 à 9.

Selon l'invention, les anticorps dirigés contre l'IGFBP-1 (anticorps anti IGFBP-1) peuvent être choisis par exemple dans le groupe comprenant les anticorps décrits dans les documents Rutanen et al, Biochem Biophys Res Commun 1988 ; 152 : 208 [5] et dans Pekonen et al, J immunoassay 1989 ; 10: 325-337 [6], les anticorps anti IGFBP-1 commercialisés par la société Lenco technologies (numéro catalogue I-695, I-746 et I-805), les anticorps anti IGFBP-1 commercialisés par la société SIGMA ALDRICH (numéro de catalogue I 2032, clone 33627.11), les anticorps commercialisés par la société AbD Serotech (numéro de catalogue 5345-4859X, clone 6302 (7B11) ; numéro de catalogue 5345-4809X, clone 7B10), les anticorps commercialisés par la société Hytest (numéro de catalogue 4152 ; clone A8 et clone G2 ; numéro de catalogue 41G8 ; clone G5F8 et clone C7B9), les anticorps commercialisés par la société Biospacific (numéro de catalogue A61403 ; numéro de catalogue A61400 ; numéro de catalogue A61402), les anticorps commercialisés par la société Santa Cruz (numéro de catalogue A61401136, A61400136, ou A61403136)

Selon l'invention, les anticorps dirigés contre l'IGFBP-1 peuvent être marqués ou non. Le marquage est exposé ci-dessous. Avantageusement, les anticorps anti IGFBP-1 utilisés en capture et les anticorps anti IGFBP-1 marqués peuvent être dirigés contre des épitopes différents.

Selon l'invention, la concentration de l'anticorps anti IGFBP-1 peut être de 0,1 à 100 µg/mL ou de 0,1 à 10 mg/ml.

Selon l'invention, le pH de solution comprenant les anticorps anti IGFPB-1 peut être de 5 à 10, de préférence de 6 à 9.

Avantageusement, selon l'invention, la concentration de chaque anticorps et/ou le pH de chaque solution comprenant chaque anticorps peut permettre de moduler le seuil de détection des protéines AFP ou IGFBP-1.

Selon l'invention, les anticorps dirigés contre AFP ou IGFBP-1 peuvent être avantageusement sous forme sèche.

Selon l'invention, les anticorps dirigés contre AFP ou IGFBP-1 peuvent être marqués par tout moyen connu de l'homme du métier. Il peut s'agir par exemple d'une molécule chimique, par exemple un marqueur fluorescent, par exemple la rhodamine ou l'isothiocyanate de fluorescéine (FITC), une enzyme par exemple la peroxydase du raifort, la phosphatase alcaline, la β-galactosidase, la glucose 6-phosphate déshydrogénase, qui est révélée en présence d' un substrat spécifique par exemple le TMB (ou 3,3',5,5'-tetramethylbenzidine), le 4-Méthyl-ombelliferyl phosphate, des nanoparticules colorées, par exemple des particules de latex ou de polystyrène, des particules d'or colloïdal, des liposomes, des particules de carbone ou de sélénium, des nanoparticules fluorescentes contenant par exemple des chélates de lanthanides (Europium par exemple), ou des nanoparticules magnétiques, des molécules radioactives, par exemple de l'iode 125, l'iode 133, le tritium.

Selon l'invention, le marquage peut être différent en fonction de la spécificité de l'anticorps. Par exemple l'anticorps anti IGFBP-1 peut être marqué avec un marqueur, différent de celui de l'anticorps anti AFP. Avantageusement, la différence de marquage permet de détecter si l'AFP seul, l'IGFBP-1 seul et/ou l'AFP et l'IGFBP-1 sont présents dans l'échantillon grâce à la différence de marquage. Les marquages différents permettent de détecter de manière distincte l'AFP et l'IGFBP-1.

Selon l'invention, les anticorps peuvent être préalablement fixés sur un support solide. Par exemple, les anticorps dirigés contre l'AFP ou l'IGFBP-1 peuvent être fixés sur une membrane, par exemple une membrane de nitrocellulose, le fond d'une plaque multi-puits et/ou toute surface connue de l'homme du métier permettant la fixation d'au moins un anticorps.

Selon l'invention la quantité d'anticorps dirigés contre l'AFP ou l'IGFBP-1 fixée peut être avantageusement adaptée en fonction de l'anticorps et de la protéine recherchée. Par exemple, lorsque l'AFP est recherchée, la quantité d'anticorps fixée peut être de 0,1 à 100 µg/mL ou de 0,1 à 10 mg/mL ; lorsque l'IGFPB-1 est recherchée, la quantité d'anticorps fixée peut être de 0,1 à 100 µg/mL ou de 0,1 à 10 mg/mL.

Le contrôle de la quantité d'anticorps fixée permet avantageusement d'adapter le seuil de détection de l'AFP et/ou l'IGFBP-1. En effet plus la quantité fixée est importante, plus le seuil de détection est faible.

En d'autres termes, selon l'invention, les anticorps peuvent être préalablement fixés sur un support, par exemple, par dépôt ou par couplage chimique.

Lorsque les anticorps sont fixés par dépôt, ils peuvent être déposés par exemple, par aspersion en une surface commune ou distincte d'anticorps anti AFP et d'anticorps anti IGFBP-1 tels que définis précédemment, puis séchage par exemple, via le procédé décrit dans Beer et al. Qualification of cellulose nitrate membranes for lateral-flow assays, IVD technology, janvier 2002. [7]

Lorsque les anticorps sont fixés par couplage chimique, ils peuvent être fixés, par exemple, via le procédé décrit dans Beer et al. Qualification of cellulose nitrate membranes for lateral-flow assays, IVD technology, janvier 2002. [7]

Selon l'invention, le procédé de l'invention peut comprendre en outre une étape préliminaire de dilution du prélèvement vaginal ou cervical dans une solution de dilution. La solution de dilution peut être toute solution connue de l'homme du métier pour diluer ces prélèvements. Il peut s'agir, par exemple, d'une solution physiologique, d'une solution tampon. Il peut s'agir par exemple aussi d'une solution comprenant 50mM de borate (02102391, Biosolve) 1%BSA (AP-4510-01, Seracare Life Sciences) 0,05% triton X-100 (pH 9,3).

Selon l'invention, le pH de la solution de dilution peut être de 5 à 10, de préférence de 6 à 9.

Selon l'invention, la force ionique de la solution de dilution peut être de 10 à 1000 mM, de préférence de 50 à 200 mM.

Selon l'invention, le procédé peut comprendre une étape de révélation.

Selon l'invention, l'étape de révélation peut être effectuée par tout procédé connu de l'homme du métier. Il peut s'agir par exemple d'un procédé radioimmunologique (RIA), immunoradiométrique (IRMA), enzymmunologique (EIA), immunofluorométrique (IFMA), immunofluorimétrique en temps résolu, luminoimmunologique (LIA), immunomagnétique, ou par immunochromatographie ou immunofiltration grâce à des particules colorées (latex, polystyrène, or colloïdal). Certains de ces procédés peuvent être mis en oeuvre comme décrit dans Anne Harwood Peruski et al. Immunological Methods for Détection and Identification of Infectious Disease and Biological Warfare Agents. Clinical and diagnostic laboratory immunology, July 2003, p. 506-513. [8]

Selon l'invention, la révélation peut être par exemple directe ou indirecte.

Lorsque la révélation est indirecte ou par compétition, on peut utiliser par exemple un anticorps marqué dirigé contre l'IGFBP-1 ou l'AFP tel que décrit précédemment.

Lorsque la révélation est directe (système sandwich), on peut utiliser par exemple un anticorps de capture et un second anticorps marqué dirigé contre l'IGFBP-1 et un anticorps de capture et un second anticorps marqué dirigé contre l'AFP tel que décrit précédemment. Avantageusement, l'anticorps de capture et l'anticorps marqué sont dirigés contre des épitopes différents de façon à former une paire utilisable en système sandwich.

De préférence, la révélation est directe.

Avantageusement, l'intensité du signal du marqueur est fonction de la quantité et/ou de la concentration d'AFP et/ou d'IGFBP-1 présente dans le prélèvement.

Suivant le marqueur utilisé, la révélation peut être visuelle ou effectuée à l'aide d'un dispositif.

Lorsque le marqueur est un marqueur coloré visible tel que décrit précédemment la révélation est, avantageusement visuelle. Lorsque le marqueur est, par exemple un marqueur radioactif tel que décrit précédemment, la révélation sera effectuée, par exemple à l'aide d'un dispositif de mesure de radioactivité, par exemple un compteur geiger.

La présente invention permet, avantageusement, de détecter l'AFP dans un prélèvement lorsque sa concentration est de 0,5 à 50 ng/mL, de 1 à 25 ng/mL, de 1 à 2 ng/mL.

La présente invention permet, avantageusement, de détecter l'AFP dans un prélèvement lorsque sa concentration est, par exemple, supérieure ou égale à une concentration comprise entre 2,5 et 50 ng/mL, de préférence comprise entre 5 à 25 ng/mL.

Dans la présente invention, la détection de l'AFP peut être réalisée dans un échantillon dilué tel que défini précédemment.

Dans la présente, la détermination du seuil de l'AFP peut être réalisée en utilisant une préparation d'AFP native provenant de la société Hytest (Ref. 8F8).

La présente invention permet, avantageusement, de détecter l'IGFBP-1 dans un prélèvement lorsque sa concentration est supérieure ou égale à une concentration comprise entre 5 et 50 ng/mL, de préférence comprise entre 10 à 30 ng/mL.

La présente invention permet également, avantageusement, de détecter l'IGFBP-1 dans un prélèvement lorsque sa concentration est supérieure ou égale à une concentration comprise entre 5 et 30 ng/mL, de préférence comprise entre 5 à 10 ng/mL.

Dans la présente invention, la détection de l'IGFBP-1 peut être réalisée dans un échantillon dilué tel que défini précédemment.

Dans la présente, la détermination du seuil de l'IGFBP-1 peut être réalisée en utilisant une préparation d'IGFBP-1 native provenant de la société Hytest (Ref. 81GB1).

Selon l'invention, le procédé de l'invention peut comprendre un ajustement d'au moins un des paramètres choisis parmi le volume du milieu tampon diluant, le pH du milieu tampon, la force ionique du milieu tampon, le dit ajustement étant commun à l'AFP et à l'IGFBP-1.

Selon l'invention, le procédé de l'invention peut comprendre un ajustement d'au moins un des paramètres choisis parmi la concentration
des anticorps de capture et/ou la concentration des anticorps marqués, le dit ajustement étant spécifique à l'AFP ou à l'IGFBP-1.

La combinaison de l'ajustement spécifique à l'AFP et à l'IGFBP-1 et/ou de l'ajustement commun permet, avantageusement, l'obtention d'une complémentarité de détection de l'AFP et de l'IGFBP-1. La complémentarité des deux marqueurs peut être, par exemple chronologique, par exemple l'AFP peut être un marqueur préférentiel avant la 35^{ème} semaine et l'IGFBP1 peut être un marqueur préférentiel au-delà de la 37^{ème} semaine. La complémentarité peut aussi concerner les performances de détection de chacun des marqueurs. Ainsi, selon la présente invention, les seuils de détection de chacun des deux marqueurs peuvent être ajustés de façon à ce que l'AFP soit le marqueur le plus sensible et l'IGFBP1 le plus spécifique et/ou réciproquement.

Selon la présente invention, il est aussi possible d'ajuster, avantageusement les paramètres précités de sorte que la spécificité de détection des marqueurs soit identique, c'est à dire qu'ils aient le même niveau de spécificité. Dans ce mode de réalisation, le marqueur IGFBP1 est statistiquement le plus sensible, notamment au-delà de la 37^{ème} semaine d'aménorrhée.

Selon l'invention, le procédé de l'invention permet avantageusement, de part la complémentarité des deux marqueurs, de détecter une rupture de membranes foetales même si l'IGFBP1 est absente, par exemple si le liquide amniotique est très peu concentré en IGFBP1 ou si l'IGFBP1 a été dégradée *in vivo* ou *in vitro* comme cela a été notamment décrit dans Rutanen et al. Measurement of Insulin-like growth factor binding protein-1 in cervical/vaginal sécrétions: comparison with the ROM-Check Membrane Immunoassay in the diagnosis of ruptured fetal membranes. Clinica Chimica Acta, 214 (1993), 73-81 [9], par la détection de l'AFP.

Selon l'invention, le procédé de l'invention permet avantageusement, de part la complémentarité des deux marqueurs, d'orienter le diagnostic vers une absence de rupture en cas de résultat positif en IGFBP1 qui serait non pas dû à une fuite de liquide amniotique mais à la présence d'IGFBP1 provenant de cellules déciduales du col. En effet, contrairement à l'IGFBP-1, l'AFP est une molécule très majoritairement d'origine foetale et par conséquent ne présente pas ce risque d'interférence. La présence d'IGFBP1 provenant des cellules déciduales du col limite l'intérêt de l'IGFBP-1; ceci a été décrit notamment dans les articles: Rutanen et al. Decidual transformation of human extrauterine mesenchymal cells is associated with is associated with the appearance of insulin like growth factor binding protein 1. J. Clin. Endocrinol. Metab. 1992 ; 72 : 27-31 [10] Rutanen et al. Evaluation of a rapid strip test for insulin-like growth factor binding protein 1 in the diagnosis of foetal ruptured membranes. Clinica Chimica Acta. 253 ; (1996) 91-101 [11]. La présente invention permet donc avantageusement de diminuer le risque de détection de faux positifs.

Selon l'invention, le procédé peut comprendre en outre une étape supplémentaire de détection seule de l'IGFBP-1.

La détection de l'IGFPB-1 seule dans l'étape supplémentaire peut être réalisée avec une sensibilité supérieure à la détection de l'IGFBP-1 lorsque effectuée simultanément avec celle de l'AFP.

Dans ce mode de réalisation, le seuil de détection est tel qu'il permet, dans le cadre de cette étape supplémentaire, de détecter l'IGFBP-1 lorsque sa concentration est de 1 à 10 ng/mL, de préférence de 2 à 5 ng/mL.

En outre dans ce mode de réalisation, la détection de l'IGFBP-1 peut être réalisée dans une solution diluée, par exemple une solution diluée telle que définie précédemment.

Dans ce mode de réalisation, les moyens de détection de l'IGFBP-1 sont ceux définis précédemment, il peut s'agir, par exemple des anticorps anti-IGFBP-1 définis précédemment.

Selon l'invention, le résultat est négatif, c'est-à-dire qu'il n'y a pas de rupture, lorsque l'on ne détecte ni l'IGFBP-1, ni l'AFP, positif, c'est-à-dire qu'il y a rupture, lorsque l'on détecte l'IGFBP1 seule ou en combinaison avec l'AFP. Lorsque seule l'AFP est détectée, le procédé de l'invention peut comprendre une étape supplémentaire de détection de l'IGFBP-1 seul tel qu'indiqué précédemment.

Avantageusement, selon ce mode de réalisation, le procédé de l'invention permet d'éliminer tous les résultats faux positifs. En outre, le procédé de l'invention dans ce mode de réalisation permet avantageusement d'obtenir une spécificité de 100% et une sensibilité supérieure à 84%.

Selon l'invention, l'échantillon peut être un échantillon prélevé à tout temps de la grossesse. Par exemple, l'échantillon peut être prélevé entre la 11^{ème} semaine et le terme de la grossesse, par exemple de 11 à 42 semaines, de 15 à 41, de 20 à 40, de 25 à 40, de 30 à 40, de 35 à 40, de 37 à 40.

Avantageusement, il est possible d'obtenir un résultat de certitude c'est-à-dire avec une sensibilité et une spécificité de 100% sur une population globale à l'exception des patientes dont le terme est supérieur à 37 à 40 semaines, par exemple 39 semaines avec AFP négatif et IGFBP-1 négatif et des patientes dont le terme est inférieur ou égal à 37 à 40 semaines, par exemple 39 semaines avec AFP positif et IGFBP-1 négatif.

Ainsi, le résultat obtenu permet de conclure à l'absence de rupture des membranes foetales sans aucun test clinique complémentaire. Dans ce mode de réalisation, le procédé de l'invention permet avantageusement d'obtenir une sensibilité et une spécificité 100% du procédé de détection.

Le procédé de l'invention permet donc d'obtenir un résultat fiable qui ne nécessite aucune étape complémentaire de confirmation et permet de conclure avec certitude quant à la rupture ou non des membranes foetale.

Le procédé de l'invention permet donc avantageusement sur une population de patientes clairement définie, c'est-à-dire dont le terme est supérieur ou inférieur à 39 semaines d'aménorrhées d'obtenir un résultat fiable qui ne nécessite aucune étape complémentaire de confirmation et permet de conclure avec certitude quant à la rupture ou non des membranes foetale.

Ce document a également pour objet un dispositif par exemple immunochromatographique pour la mise en oeuvre du procédé selon l'invention comprenant :
- une zone (1) de dépôt d'un échantillon
- une zone (2) comprenant des anticorps anti IGFBP-1 et des anticorps anti AFP marqués.
- une zone (3) de révélation comprenant des anticorps de capture anti AFP et des anticorps de capture anti IGFPB-1.

La présente invention a également pour objet l'utilisation du dispositif comprenant : a. une zone (1) de dépôt d'un échantillon b. une zone (2) comprenant des anticorps anti IGFBP-1 et des anticorps anti AFP marqués. c. une zone (3) de révélation comprenant des anticorps de capture dirigé contre AFP et des anticorps de capture dirigé contre l'IGFPB-1 pour la mise en oeuvre du procédé de détection in-vitro d'une rupture de membranes foetales selon l'une quelconque des revendications 1 à 9.

Selon l'invention, la zone (1) de dépôt peut être une zone adaptée à l'application ou la réception du prélèvement. Cette zone peut être de toute forme connue de l'homme du métier, par exemple un réservoir, une cupule, un puits, une mèche, une surface plane

Selon l'invention la zone (1) de dépôt peut être mobile et/ou liée à la zone (2) ou (3). Lorsque la zone (1) est mobile, elle peut être, par exemple, utilisée pour effectuer le prélèvement et appliquée sur la zone (2). Lorsque la zone (1) est liée à la zone (2) ou (3) celle-ci peut être plongée directement dans un récipient comprenant le prélèvement et/ou le prélèvement peut être appliqué sur cette zone. Un exemple de ce dispositif est illustré ci-dessous.

Avantageusement, selon l'invention, les zones (1) et (2) sont situées sur un même support.

Selon l'invention, les matériaux des zones (1) à (3) peuvent être identiques ou différents. Les matériaux peuvent être tout matériau connu de l'homme du métier, par exemple un matériau choisi dans le groupe comprenant du papier absorbant, du coton, de la cellulose, de la fibre de verre, une membrane de nitrocellulose.

De préférence, la zone de dépôt (1) peut être un papier absorbant ou de la fibre de verre.

De préférence, la zone (2) comprenant des anticorps anti IGFBP-1 et des anticorps anti AFP marqués peut être de la fibre de verre.

De préférence, la zone de révélation (3) peut être une membrane de nitrocellulose.

Selon l'invention, les anticorps de capture anti AFP ou anti IGFBP-1 peuvent être, par exemple fixés directement sur le support de la zone (3), par exemple par couplage chimique et/ou par dépôt tel que décrit précédemment.

Selon l'invention, la zone de fixation de l'anticorps de capture de l'AFP et de l'anticorps de capture de l'IGFBP-1 peut être identique ou différente. De préférence, la zone de fixation de l'anticorps de capture de l'AFP est différente de celle de l'anticorps de capture de l'IGFBP-1. Il peut s'agir par exemple de deux bandes parallèles comprenant chacune un anticorps de capture différent.

Selon l'invention les différentes zones (1) à (3) précitées peuvent être fixées sur un support solide, par exemple sur des cartes laminées et/ou comprises dans un contenant comprenant un orifice au niveau de la zone (3) permettant la visualisation du résultat et un orifice au niveau de la zone (1) de dépôt de l'échantillon permettant le dépôt de l'échantillon. Un exemple de ce dispositif est illustré ci-dessous.

Selon l'invention, le dispositif peut comprendre en outre une zone (4) d'absorption. Selon l'invention, la zone d'absorption (4) peut être n'importe quel support solide absorbant connu de l'homme du métier. Il peut s'agir, par exemple, d'un papier buvard absorbant, d'une éponge, d'un coton, d'une feutrine ou d'un textile synthétique.

Avantageusement, selon l'invention, les supports des zones (1) et (2) se chevauchent à l'une de leur extrémité et l'autre extrémité de la zone (2) se chevauche avec une extrémité de la zone (3). Le chevauchement des différentes zones (1) à (3) permet avantageusement lors de l'application du prélèvement et/ou de l'immersion de l'extrémité libre de la zone (1) dans le prélèvement la migration par capillarité du prélèvement dans les différentes zones. De préférence, les zones (1) et (2) sont disposées sur un même support. Un exemple de ce dispositif est illustré ci-dessous.

De préférence, la zone (4) d'absorption se chevauche avec l'extrémité libre de la zone (3). Ainsi, la zone (4) permet une migration accélérée du prélèvement à travers les différentes zones du dispositif. Avantageusement, la zone (4) permet d'absorber l'excédent de liquide du prélèvement.

Selon l'invention, les différentes zones (1) à (4) peuvent être indépendamment recouvertes avec un film de protection. Il peut s'agir ; par exemple d'un film plastique, par exemple un film en chlorure de polyvinyle (PVC), un film biodégradable, par exemple un film en Polycaprolactone (PCL), en alcool Polyvinyle (PVA), en acide Polylactique (PLA).

Le film permet avantageusement de protéger indépendamment les différentes zones du dispositif de l'invention.

Selon l'invention, le film peut être un film unique ou plusieurs films recouvrant indépendamment une des dites zones (1) à (4).

Selon l'invention, le film ou les films peuvent recouvrir partiellement le dispositif de l'invention laissant ainsi certaines zones non couvertes. Selon l'invention, le film ou les films peut (peuvent) être un (des) film(s) détachable(s) qui peut (peuvent) être enlevé(s) avant l'utilisation du dispositif. Selon l'invention, le film peut comprendre une inscription à sa surface permettant avantageusement une identification du dispositif.

Ce document a également pour objet une trousse pour la mise en oeuvre du procédé de l'invention comprenant :
- des anticorps de capture anti AFP et anti IGFBP-1 tels que définis précédemment
- des anticorps marqués anti AFP et anti IGFP1 tels que définis précédemment.

La trousse peut comprendre en outre un moyen de prélèvement, par exemple un écouvillon, tel que défini précédemment.

La trousse peut comprendre en outre un support solide sur lequel peuvent être fixés des anticorps de capture tels que définis précédemment.

La présente invention permet avantageusement de détecter des ruptures de membranes foetales quelle que soit leur importance, par exemple s'il s'agit de microruptures et/ou de macroruptures et/ou quelque soit le volume de liquide amniotique prélevé.

La présente invention permet, avantageusement, de diminuer la probabilité d'obtenir des résultats faux négatifs ainsi que des résultats faux positifs.

En outre, la présente invention permet avantageusement de détecter des ruptures de membranes foetales quelque soit le stade de la grossesse.

La présente invention permet en outre une détection fiable quels que soient le stade de la grossesse, la variation de la concentration de l'AFP et/ou de l'IGFBP-1 dans le liquide amniotique et/ou le volume de liquide amniotique présent dans le prélèvement vaginal ou cervical.

D'autres avantages pourront encore apparaître à l'homme du métier à la lecture des exemples ci-dessous, illustrés par les figures annexées, donnés à titre illustratif.

### Brève description des figures

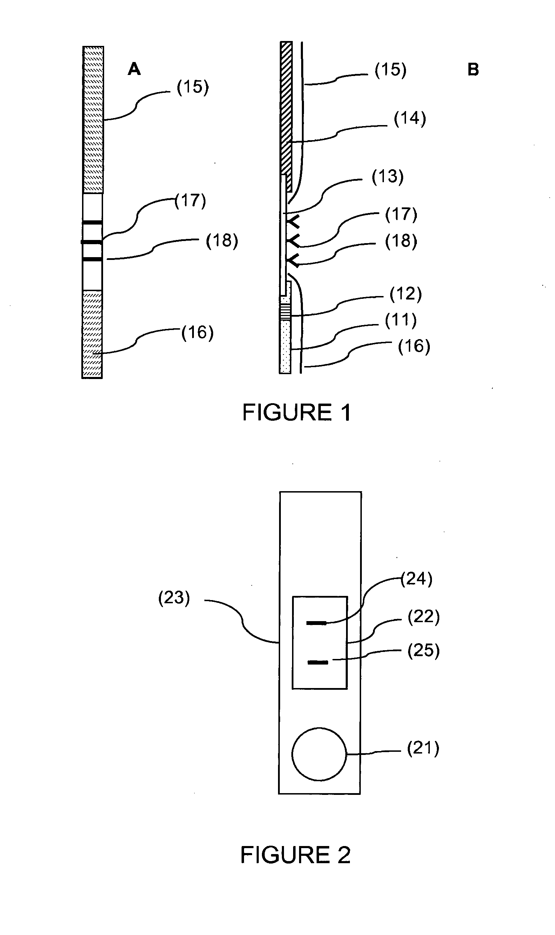

- La figure 1 représente une vue de face (A) et une vue de profil d'un dispositif selon l'invention comprenant une zone (1) en fibre de verre(11), une zone (2) en fibre de verre (12) comprenant des anticorps anti IGFBP-1 et des anticorps anti AFP marqués (12), une zone (3) en membrane de nitrocellulose (13) comprenant des anticorps de capture anti AFP (17) et des anticorps de capture anti IGFPB-1 (18) et une zone (4) en papier absorbant (14), un film (15) (16)
- La figure 2 représente un dispositif compris dans un contenant (23) comprenant un orifice (21) au niveau de la zone (1) et un orifice (22) au nouveau de la zone (3) comprenant des anticorps de capture anti AFP (24) et des anticorps de capture anti IGFPB-1 (25).

### EXEMPLES

### Exemple 1 : procédé de détection de rupture de membranes foetales grâce à un dispositif d'immunofiltration.

Des anticorps anti AFP (ABAFP-0404, clone 4 Arista Biologicals) dilués à 0,75 mg/mL dans un tampon PBS ainsi que des anticorps anti IGFBP-1 (I 2032, clone 33627.11, SIGMA ALDRICH) dilués à 0,75 mg/mL dans un tampon PBS sont préparés.

Les anticorps sont déposés à l'aide d'un automate de distribution de réactifs (Isoflow Dispenser, Imagene Technology Inc. (marque déposée)) sous forme de lignes parallèles sur une membrane de nitrocellulose puis séchés à l'étuve à 37°C sous atmosphère à taux d'humidité contrôlé inférieur à 30%. La membrane est ensuite découpée en multiples bandelettes d'environ 1 centimètre de large et 2,5 centimètre de long. Chaque bandelette est ensuite placée sur un support absorbant posé dans un socle en plastique. Un couvercle en plastique s'adaptant au socle est ensuite posé sur le socle et fermé par pression manuelle. Le couvercle en plastique dispose d'une fenêtre d'ouverture dont la surface est inférieure à la surface de la membrane. La membrane a été positionnée de sorte que la zone ouverte de la fenêtre du couvercle soit totalement située au dessus de la membrane. Le couvercle est conçu de façon à ce qu'il exerce une pression sur la membrane et le support absorbant afin que les différents éléments du dispositif gardent la position définie lors de la fermeture du couvercle.

Un prélèvement avec un écouvillon stérile dont le bouton est du polyester est effectué sur une femme enceinte à 24 semaines d'aménorrhées.

Le bouton est ensuite introduit dans un tube à vis de 5 mL (Dustcher) comprenant 2 mL d'une solution d'extraction contenant 50mM de borate (02102391, Biosolve) 1% Sérum Albumine de Bovin (BSA) (AP-4510-01, Seracare Life Sciences) 0,05% triton X-100 (pH 9,3) et est agité par mouvements circulaires pendant 30 secondes à une minute. L'écouvillon est ensuite retiré du tube.

La solution d'extraction est ensuite versée dans la fenêtre du couvercle. La solution est filtrée à travers la membrane et absorbée par le support absorbant. L'AFP et/ou l'IGFBP-1 contenu(s) dans la solution d'extraction se lie(nt) avec les anticorps de capture respectifs. Lors de l'étape suivante, un réactif de révélation contenant des anticorps anti AFP le clone 3, Arista Biologicals, ABAFP-0403, et anti IGFBP-1, le clone 6302 (7B11), AbD Serotech, 5345-4859X, de paratopes différents des anticorps de capture et marqués à l'or colloïdal, se lie aux antigènes AFP et/ou IGFBP-1 capturés. Cela entraîne l'apparition d'une coloration mauve au niveau des lignes tests. Une étape de lavage avec une solution de lavage contenant par exemple du PBS 10% TWEEN-20 est réalisée par versement de la solution de lavage dans la fenêtre pour éliminer toute fixation non spécifique des particules d'or colloïdal. La présence de lignes colorées mauves au niveau des lignes AFP et/ou IGFBP-1 est indicatrice de rupture des membranes foetale.

### Exemple 2 : Dispositif immunochromatographique pour la détection de rupture de membranes foetale.

Les bandelettes sont préparées à partir de cartes laminées de 30 cm X 9,5 cm (CNPC-SS12 R-032/2, MDI (marque déposée)) constituées d'un support en matière plastique recouvert d'une couche d'adhésif sur laquelle la membrane de nitrocellulose, le papier absorbant et la fibre de verre sont assemblés.

A l'aide d'un automate de distribution de réactifs (Isoflow Dispenser, Imagene Technology Inc. (marque déposée)), des anticorps de chèvre anti IgG de souris (ABGAM-0500, Arista Biologicals (marque déposée)) dilués à 4 mg/mL dans un tampon PBS, des anticorps anti AFP (ABAFP-0404, clone 4 Arista Biologicals) dilués à 0,75 mg/mL dans un tampon PBS ainsi que des anticorps anti IGFBP-1 (I 2032, clone 33627.11, SIGMA ALDRICH (marque déposée)) dilués à 0,75 mg/mL dans un tampon PBS sont déposés avec un débit de 0,5 µl/cm sous forme de lignes parallèles espacées de 5 mm et d'une largeur de 1 à 2 mm chacune sur une membrane de nitrocellulose dont la largeur est de 25 mm (zone (1)).

Après dépôt de ces moyens de recherche, ces cartes sont ensuite placées 30 minutes dans une étuve à 37°C sous atmosphère à taux d'humidité contrôlé inférieur à 30% pour les sécher.

Le mélange de conjugué, c'est-à-dire une solution comprenant des anticorps marqués, est préparé à partir des anticorps anti IGFBP-1 (5345-4859X, clone 6302 (7B11), AbD Serotech (marque déposée)) et anti-AFP (ABAFP-0403, clone 3, Arista Biologicals (marque déposée)) préalablement couplés à des particules de latex (DBK1040CB, diamètre 0.39µm, Duke Scientific Corporation (marque déposée)) selon les indications du fournisseur ou les instructions suivantes :
Les particules de latex (150µL) sont lavées deux fois avec 1 mL de PBS par centrifugation pendant 30 minutes à 14000 tours par minute (rpm) dans des tubes eppendorf (marque déposée) de 1,5 mL. A la dernière étape de lavage, les particules de latex sont remises en suspension dans la même solution. Les parties carboxylées des billes sont ensuite activées par 50 µg de N-(3-Dimethylaminopropyl)-N-ethylcarbodiimide hydro-chloride (EDC, E1769, SIGMA-ALDRICH (marque déposée)). Après 15 minutes d'activation, la solution d'anticorps est ajoutée à raison de 10µg/mg de particules de latex. La solution ainsi obtenue est délicatement agitée pendant 2h à température ambiante sur un agitateur circulaire adapté aux tubes de 1,5 mL. Une étape de centrifugation pendant 15 minutes à 14000 tours par minute permet ensuite de récupérer les particules de latex conjuguées aux anticorps. Afin d'éviter les interactions non spécifiques, le culot est repris dans 1 mL d'une solution de saturation (pH 7,4) 50mM Na₂HPO₄ (S7907, SIGMA ALDRICH (marque déposée)) 1% BSA (AP-4510-01, Seracare Life Sciences (marque déposée)). La solution est agitée sur l'agitateur circulaire 1 heure à température ambiante puis placée dans une étuve pendant 30 minutes à 37°C. La solution est centrifugée une dernière fois pendant 15 minutes à 14000 tours par minute. Le culot ainsi obtenu est remis en suspension dans la même solution de saturation.

A 250µL de solution de conjugué IGFBP-1 (anticorps anti IGFBP-1 marqués) additionnés de 250µL de conjugué AFP (anticorps anti AFP marqués) sont ajoutés un volume de la solution de saturation (pH 7,4) 50mM Na₂HPO₄ (S7907, SIGMA ALDRICH (marque déposée)) 1% de d'Albumine de sérum de bovin (BSA) (AP-4510-01, Seracare Life Sciences) qsp 1 mL, 20% de sucrose (S8501, SIGMA ALDRICH) et 5% de tréhalose (T9531, SIGMA ALDRICH (marque déposée)). Le mélange ainsi obtenu est agité 15 minutes à température ambiante sur l'agitateur circulaire adapté aux tubes de 1,5 mL.

Les anticorps ont été ainsi couplés avec les dites particules de latex et correspondent à des exemples d'anticorps marqués.

Le mélange d'anticorps couplés aux microparticules de latex est ensuite vaporisé avec un débit de 4 µl/cm sur des feuilles de fibre (zone (3)) de verre de dimension 30 cm X 3,2 cm (# 8964, AHLSTROM, Arista Biologicals (marque déposée)) préalablement traitées avec une solution 0,1 M de B₄Na₂O₇ (B3545, SIGMA ALDRICH (marque déposée)) 1% triton X-100 (X-100, SIGMA ALDRICH (marque déposée)) pH 8,4.

La fixation de ce mélange de conjugués sur la feuille de fibre de verre est réalisée avec le même appareil Isoflow Dispenser (marque déposée) en se conformant aux indications du fabricant. Après vaporisation, les feuilles de fibre de verre sont séchées une demi-heure à 37°C.

L'assemblage des cartes se fait par collage de la fibre de verre imprégnée de conjugué sur la partie adhésive inférieure de la carte en faisant chevaucher la feuille de fibre de verre de 1 à 3 mm sur la membrane de nitrocellulose. De la même manière, un papier de haut pouvoir d'absorption (zone 2) (absorbant pad, Ahlstrom 222, MAPDS-0100, Arista Biologicals) de dimension 30 cm X 4,2 cm est placé sur la partie supérieure de la carte et chevauche la membrane de nitrocellulose de 1 à 3 mm afin de pouvoir créer un flux de migration. Une étiquette autocollante est alors déposée sur les deux sortes de papier et ont pour fonction de protéger et d'identifier les bandelettes.

La carte ainsi assemblée est découpée en bandelettes de 4 mm de largeur à l'aide d'une guillotine (GCI-800, Zeta Corporation) ou d'un appareil à multi lames circulaire (Rotary cutter, Arista Biologicals Inc). Une bandelette est représentée par le schéma ci-dessous :

### Exemple 3 : Exemple d'utilisation d'un dispositif pour mettre en oeuvre le procédé de l'invention

1. Dans un tube à vis de 5 mL (015610, Dustcher), une solution de 2,5 mL de 50mM de borate (02102391, Biosolve) 1% BSA (AP-4510-01, Seracare Life Sciences) 0,05% triton X-100 (pH 9,3) a été introduite,
2. l'échantillon a été prélevé avec un écouvillon stérile dont le bouton est du polyester et déposé sur le dispositif décrit dans l'exemple 2,
3. le dispositif obtenu a été introduit dans le tube à vis précité
4. le résultat est obtenu après 10 minutes de migration

Le test est positif lorsqu'au moins une des deux bandes correspondant à l'AFP et à l'IGBPF-1 est colorée.

Lorsque que le test est positif, la véracité du test peut être vérifiée par un examen gynécologique complet permettant de confirmer ou d'infirmer le résultat.

### Exemple 4 : comparaison de la stabilité aux protéases de l'AFP et de l'IGFBP-1 réalisée à l'aide d'un dispositif mettant en oeuvre le procédé de l'invention.

Dans cet exemple le dispositif immunochromatographique décrit dans l'exemple 2 est modifié et utilise un système de marquage à l'or colloïdal pour la détection des molécules d'IGFBP-1 et d'AFP. Les paires d'anticorps anti IGFBP-1 et anti AFP ont été sélectionnées dans la liste d'anticorps présentés dans la description de l'invention.

Deux microlitres et dix microlitres d'un liquide amniotique sont déposés dans deux tubes distincts contenant chacun 2,5 millilitres de solution d'extraction. Puis 200 microlitres d'urines fraiches sont ajoutés à chacun des deux tubes. Les bandelettes sont ensuite déposées dans les solutions d'extraction selon les modalités décrites dans l'exemple 3 ci-dessus. Les tubes contenant la solution d'extraction sont ensuite placés à 37°C et sont testés à 24 heures, 48 heures et 6 jours. L'échantillon de liquide amniotique a été sélectionné par sa propriété de générer des signaux de départ (au temps T0) d'intensité identique sur les bandes AFP et IGFBP-1.

Sur le tube contenant deux microlitres de liquide amniotique, après 24 heures d'incubation, le signal IGFBP-1 a baissé de façon significative tandis que le signal AFP n'a que très légèrement baissé. Après 48 heures d'incubation, le signal IGFBP-1 a disparu alors que l'intensité du signal AFP n'a pas évolué par rapport à la lecture précédente de 24 heures. A 6 jours, le signal AFP reste visible et inchangé tandis que le signal IGFBP-1 reste absent.

Sur le tube contenant dix microlitres de liquide amniotique, après 24 heures d'incubation, les signaux IGFBP-1 et AFP restent inchangés. Après 48 heures d'incubation, le signal IGFBP-1 a baissé alors que le signal AFP n'a pas évolué. A 6 jours, le signal AFP reste inchangé tandis que le signal IGFBP-1 s'est très fortement atténué et n'est plus apparent que sous forme de trace.

### Exemple 5: comparaison des performances de l'association AFP/IGFBP1 vs IGFBP-1 seul

Une étude prospective a été menée du 17 novembre 2010 au 15 janvier 2011 parmi un groupe de 102 patientes. Ont été inclues toutes les patientes qui ont consulté pour suspicion d'écoulement de liquide amniotique au-delà de 12 semaines d'aménorrhée (SA) et pour lesquelles le clinicien, après examen clinique sous spéculum, retenait une indication à réaliser un test biologique de recherche de liquide amniotique. Les patientes présentant des métrorragies n'étaient pas éligibles. Le prélèvement consistait à collecter les sécrétions présentes au niveau du cul de sac vaginal avec deux écouvillons en parallèle : l'écouvillon stérile en polyester (25-806, Pur-Wraps (marque déposée) Puritan) utilisé avec le dispositif mettant en oeuvre le procédé de l'invention et l'écouvillon ACTIM^{®} (marque déposée) PROM (25-806, Pur-Wraps (marque déposée) Puritan) utilisé avec le test ACTIM PROM.

ACTIM^{®} (marque déposée) PROM (Medix Biochemica (marque déposée)) est un test immunochromatographique qui utilise deux anticorps dirigés contre l'IGFBP-1 humaine. L'un est fixé sur des particules de latex bleues et l'autre est immobilisé sur la membrane de la bandelette formant une ligne de capture. L'écouvillon est plongé dans 0,5 mL de la solution d'extraction fournie dans le kit (solution tamponnée au phosphate contenant de l'albumine bovine sérique, des inhibiteurs de protéase ainsi que des conservateurs). Il est agité vigoureusement pendant 10 secondes. La bandelette est immergée jusqu'à ce que le front de liquide arrive au niveau de la zone de résultats puis elle est retirée. La lecture se fait après 5 minutes de migration.

Un résultat est considéré positif si deux lignes bleues apparaissent ce qui traduit la présence d'IGFBP-1 à un taux supérieur à 25µg/mL.

Le dispositif mettant en oeuvre le procédé de l'invention est un test immunochromatographique qui utilise un système de marquage à l'or colloïdal d'anticorps anti-AFP et anti-IGFBP-1 et d'immobilisation d'anticorps AFP et IGFBP-1 sur la membrane de la bandelette pour la détection des molécules d'IGFBP-1 et d'AFP sur deux lignes distinctes. Dans cet exemple, l'écouvillon a été plongé pendant 10 secondes dans un tube à vis de 2 mL (015610, Dustcher) avec 0,5 mL de solution 50mM de NA₂HPO₄ (P4417,Sigma Aldrich) 1% BSA (AP-4510-01, Seracare Life Sciences) 0,05% triton (X-100, Sigma aldrich) pH 7,4. Ce prélèvement dilué a été conservé à -20°C jusqu'à ce que l'ensemble des échantillons soient récoltés. Lors de la réalisation du test, le dispositif mettant en oeuvre le procédé de l'invention a été introduit dans le tube à vis précité. Le résultat a été lu après 10 minutes de migration.

Les bandelettes ont été préparées à partir de cartes laminées de 30 cm X 9,5 cm (CNPC-SS12 R-032/2, MDI (marque déposée)) constituées d'un support en matière plastique recouvert d'une couche d'adhésif sur laquelle la membrane de nitrocellulose, le papier absorbant et la fibre de verre ont été assemblés.

A l'aide d'un automate de distribution de réactifs (Isoflow Dispenser, Imagene Technology Inc. (marque déposée)), des anticorps de chèvre anti IgG de souris (ABGAM-0500, Arista Biologicals (marque déposée)) dilués à 2 mg/mL dans un tampon PBS pH 7,4 NA₂HPO₄ 0,015M (P4417, Sigma (marque déposée)) NaCl 0,15M (S7653, Sigma (marque déposée)), des anticorps anti-AFP qui ont été choisis dans la liste suivante : société Anticorps en Ligne (numéro de catalogue ABIN93624), les anticorps commercialisés par la société Arista Biologicals (clone 1, numéro de catalogue ABAFP-0401 ; clone 2, numéro de catalogue ABAFP-0402 ; clone 3, numéro de catalogue ABAFP-0403 ; clone 4, numéro de catalogue ABAFP-0404), les anticorps commercialisés par la société Hytest (clone 5H7, clone 4A3 et clone XA2, numéro de catalogue 4F16 ; anticorps polyclonal, numéro de catalogue ABAFP-0501) dilués à 1 mg/mL dans un tampon PBS ainsi que des anticorps anti-IGFBP-1 qui ont été choisis dans la liste suivante : les anticorps anti IGFBP-1 commercialisés par la société Lenco technologies (numéro catalogue I-695, I-746 et I-805), les anticorps anti IGFBP-1 commercialisés par la société SIGMA ALDRICH (numéro de catalogue I 2032, clone 33627.11), les anticorps commercialisés par la société AbD Serotech (numéro de catalogue 5345-4859X, clone 6302 (7B11); numéro de catalogue 5345-4809X, clone 7B10), les anticorps commercialisés par la société Hytest (numéro de catalogue 4152 ; clone A8 et clone G2 ; numéro de catalogue 4IG8 ; clone G5F8 et clone C7B9) dilués à 0,5 mg/mL dans un tampon PBS ont été déposés avec un débit de 1 µl/cm sous forme de lignes parallèles espacées de 5 mm et d'une largeur de 1 à 2 mm chacune, sur une membrane de nitrocellulose dont la largeur est de 25 mm (zone (1)).

Après dépôt de ces moyens de recherche, ces cartes ont été ensuite placées 30 minutes dans une étuve à 37°C sous atmosphère à taux d'humidité contrôlé inférieur à 30% pour les sécher.

Le mélange de conjugué, c'est-à-dire une solution comprenant des anticorps marqués, a été préparé à partir des anticorps anti IGFBP-1 qui ont été choisis dans la liste suivante : les anticorps anti IGFBP-1 commercialisés par la société Lenco technologies (numéro catalogue I-695, I-746 et I-805), les anticorps anti IGFBP-1 commercialisés par la société SIGMA ALDRICH (numéro de catalogue I 2032, clone 33627.11), les anticorps commercialisés par la société AbD Serotech (numéro de catalogue 5345-4859X, clone 6302 (7B11); numéro de catalogue 5345-4809X, clone 7B10), les anticorps commercialisés par la société Hytest (numéro de catalogue 4152 ; clone A8 et clone G2 ; numéro de catalogue 4IG8 ; clone G5F8 et clone C7B9) et anti-AFP qui ont été choisis dans la liste suivante: (société Anticorps en Ligne (numéro de catalogue ABIN93624), les anticorps commercialisés par la société Arista Biologicals (clone 1, numéro de catalogue ABAFP-0401 ; clone 2, numéro de catalogue ABAFP-0402 ; clone 3, numéro de catalogue ABAFP-0403 ; clone 4, numéro de catalogue ABAFP-0404), les anticorps commercialisés par la société Hytest (clone 5H7, clone 4A3 et clone XA2, numéro de catalogue 4F16 ; anticorps polyclonal, numéro de catalogue ABAFP-0501) préalablement couplés à des particules d'or colloïdal selon le protocole suivant :

A 100mL d'eau distillée 1,0 mL de solution de chlorure d'or 1 % a été ajouté (G4022, Sigma Aldrich). L'eau a été chauffée jusqu'au point d'ébullition et 2,5mL de solution de citrate de sodium 1% (S1804, Sigma Aldrich) ont été ajoutés. La solution est devenue incolore puis a viré au pourpre. Au bout d'une minute, la solution a été chauffée jusqu'à ce qu'elle devienne rouge cerise. 9,5mL de citrate de sodium 1% (S1804, Sigma Aldrich) ont été ajoutés. L'eau a été chauffée jusqu'au point d'ébullition et 8,6mL de solution de chlorure d'or 1% (G4022, Sigma Aldrich) ont été ajoutés. La solution est devenue bleue foncée quasi noire, puis pourpre puis a été laissée refroidir à température ambiante.

Le pH a été ajusté, d'une part, à l'aide d'une solution de carbonate de potassium 0,2M (269619, Sigma Aldrich) de 10mL de la solution d'or colloïdal pourpre préalablement obtenue jusqu'à atteindre pH 7,8 ; et d'autre part, ajusté à l'aide d'une solution de carbonate de potassium 0,2M (269619, Sigma Aldrich) de 10 mL de solution d'or colloïdal pourpre à pH 7,3. 200µg d'anti-IGFBP1 ont été ajoutés à 1 mL d'eau distillée puis 10mL de solution d'or colloïdal pourpre au pH ajusté à 7,8 ont été ajoutés rapidement. 200µg d'anti-AFP ont été ajoutés à 1 mL d'eau distillée puis 10mL de solution d'or colloïdal pourpre au pH ajusté à 7,3 ont été ajoutés rapidement. Ces tubes ont été placés sur agitateur circulaire (SB2, STUART (marque déposée)) pendant 20 minutes. 1 mL de solution BSA 10% (AP-4510-01, Seracare Life Sciences (marque déposée)) et 50µL de la solution de PEG 10% (81275, Sigma Aldrich (marque déposée)) ont été ajoutés. Les 2 tubes ont été centrifugés (5804, Eppendorf (marque déposée)) à 4000 rcf pendant 30 minutes. Le surnageant a été aspiré à l'aide d'une pompe à vide (159600, BRAND (marque déposée)) et le culot a été repris avec 1 mL de tampon de resuspension (pH 8,0) 20mM TRIS BASE (26-128-3094, Euromedex (marque déposée)), 50mM NaCl (S7653, Sigma Aldrich (marque déposée)), 0,2% BSA (AP-4510-01, Seracare Life Sciences (marque déposée)), 10% sucrose (S8501, Sigma Aldrich), 5% tréhalose (T9531, Sigma Aldrich (marque déposée)).

A 250µL de solution de conjugué IGFBP-1 additionnés de 250µL de conjugué AFP ont été ajoutés 500µL de la solution de saturation (pH 7,4) 50mM Na₂HPO₄ (S7907, SIGMA ALDRICH (marque déposée)) 1% de d'Albumine de sérum de bovin (BSA) (AP-4510-01, Seracare Life Sciences), 20% de sucrose (S8501, SIGMA ALDRICH) et 5% de tréhalose (T9531, SIGMA ALDRICH (marque déposée)). Le mélange ainsi obtenu a été agité 15 minutes à température ambiante sur l'agitateur circulaire adapté aux tubes de 1,5 mL.

Les anticorps ont été ainsi couplés avec les dites particules d'or colloïdal et correspondent à des exemples d'anticorps marqués.

Le mélange d'anticorps couplés aux microparticules d'or a été ensuite vaporisé avec un débit de 4 µl/cm sur des feuilles de fibre de verre (zone (3)) de dimension 30 cm X 3,2 cm (# 8964, AHLSTROM, Arista Biologicals (marque déposée)) préalablement traitées avec une solution 0,1 M de B₄Na₂O₇(B3545, SIGMA ALDRICH (marque déposée)) 1% triton X-100 (X-100, SIGMA ALDRICH (marque déposée)) pH 8,4.

La fixation de ce mélange de conjugués sur la feuille de fibre de verre a été réalisée avec le même appareil Isoflow Dispenser (marque déposée) en se conformant aux indications du fabricant. Après vaporisation, les feuilles de fibre de verre ont été séchées une demi-heure à 37°C.

L'assemblage des cartes a été réalisé par collage de la fibre de verre imprégnée de conjugué sur la partie adhésive inférieure de la carte en faisant chevaucher la feuille de fibre de verre de 1 à 3 mm sur la membrane de nitrocellulose. De la même manière, un papier à grand pouvoir d'absorption (zone 2) (absorbant pad, Ahlstrom 222, MAPDS-0100, Arista Biologicals) de dimension 30 cm X 4,2 cm a été placé sur la partie supérieure de la carte et chevauche la membrane de nitrocellulose de 1 à 3 mm afin de pouvoir créer un flux de migration. Une étiquette autocollante a été alors déposée sur les deux sortes de papier et ont pour fonction de protéger et d'identifier les bandelettes.

La carte ainsi assemblée a été découpée en bandelettes de 4 mm de largeur à l'aide d'une guillotine (GCI-800, Zeta Corporation) ou d'un appareil à multi lames circulaire (Rotary cutter, Arista Biologicals Inc).

Le tableau 1 ci-dessous détaille des exemples de résultats obtenus ainsi que les interprétations correspondantes par les obstétriciens :

| **N° échantillon** | **TERME** | **Procédé de l'état de la technique** | **Dispositif de l'invention** | | | **Avis Obstétricien** |
|---|---|---|---|---|---|---|
| | | **INTERPRETATION** | **IGFBP-1** | **AFP** | **INTERPRETATION** | |
| 101 | 27 SA | NEGATIF | NEGATIF | NEGATIF | NEGATIF | **NEGATIF** |
| 103 | 35 SA | POSITIF | POSITIF | POSITIF | POSITIF | **POSITIF** |
| 104 | NC | NEGATIF | NEGATIF | NEGATIF | NEGATIF | **NEGATIF** |
| 105 | 28 SA | NEGATIF | NEGATIF | NEGATIF | NEGATIF | **NEGATIF** |
| 106 | 26 SA | NEGATIF | NEGATIF | NEGATIF | NEGATIF | **NEGATIF** |
| 107 | 39 SA | POSITIF | POSITIF | POSITIF | POSITIF | **POSITIF** |
| 109 | 41 SA | NEGATIF | NEGATIF | NEGATIF | NEGATIF | **NEGATIF** |
| 111 | 40 SA | NEGATIF | NEGATIF | NEGATIF | NEGATIF | **NEGATIF** |
| 112 | 40SA+4j | POSITIF | POSITIF | NEGATIF | POSITIF | **POSITIF** |
| 113 | 30 SA | NEGATIF | NEGATIF | NEGATIF | NEGATIF | **NEGATIF** |
| 114 | 36 SA | POSITIF | POSITIF | POSITIF | POSITIF | **POSITIF** |
| 115 | 34SA+4j | NEGATIF | POSITIF | POSITIF | POSITIF | **POSITIF** |
| 116 | 22 SA | POSITIF | POSITIF | POSITIF | POSITIF | **POSITIF** |
| 117 | 26 SA | POSITIF | POSITIF | POSITIF | POSITIF | **POSITIF** |
| 118 | 40 SA | POSITIF | POSITIF | POSITIF | POSITIF | **POSITIF** |
| 119 | 40 SA+2j | POSITIF | NEGATIF | NEGATIF | NEGATIF | **NEGATIF** |
| 120 | 40 SA | POSITIF | POSITIF | POSITIF | POSITIF | **POSITIF** |
| 122 | 36 SA | POSITIF | POSITIF | POSITIF | POSITIF | **POSITIF** |
| 123 | 39 SA | NEGATIF | NEGATIF | NEGATIF | NEGATIF | **NEGATIF** |
| 124 | 37 SA | NEGATIF | NEGATIF | NEGATIF | NEGATIF | **NEGATIF** |
| 125 | 41 SA | NEGATIF | NEGATIF | NEGATIF | NEGATIF | **NEGATIF** |
| 126 | 39SA+2j | POSITIF | POSITIF | NEGATIF | POSITIF | **POSITIF** |
| 129 | 30 SA | NEGATIF | NEGATIF | NEGATIF | NEGATIF | **NEGATIF** |
| 130 | 33 SA | NEGATIF | NEGATIF | NEGATIF | NEGATIF | **NEGATIF** |
| 131 | 12 SA | NEGATIF | NEGATIF | NEGATIF | NEGATIF | **NEGATIF** |
| 132 | 37 SA | NEGATIF | NEGATIF | NEGATIF | NEGATIF | **NEGATIF** |
| 133 | 31 SA | NEGATIF | NEGATIF | NEGATIF | NEGATIF | **NEGATIF** |
| 134 | 38 SA | NEGATIF | NEGATIF | NEGATIF | NEGATIF | **NEGATIF** |
| 135 | 33 SA | NEGATIF | NEGATIF | NEGATIF | NEGATIF | **NEGATIF** |
| 136 | 41SA+4j | POSITIF | POSITIF | NEGATIF | POSITIF | **POSITIF** |
| 137 | 37SA+6j | POSITIF | POSITIF | NEGATIF | POSITIF | **POSITIF** |
| 139 | 30 SA | NEGATIF | NEGATIF | NEGATIF | NEGATIF | **NEGATIF** |
| 140 | 41 SA | NEGATIF | NEGATIF | NEGATIF | NEGATIF | **NEGATIF** |
| 141 | 41 SA | NEGATIF | NEGATIF | NEGATIF | NEGATIF | **POSITIF** |
| 143 | 40 SA | POSITIF | POSITIF | POSITIF | POSITIF | **POSITIF** |
| 144 | 40 SA | NEGATIF | NEGATIF | NEGATIF | NEGATIF | **NEGATIF** |
| 145 | 23 SA | NEGATIF | NEGATIF | NEGATIF | NEGATIF | **NEGATIF** |
| 146 | 30 SA | NEGATIF | NEGATIF | NEGATIF | NEGATIF | **NEGATIF** |
| 147 | 40 SA | POSITIF | POSITIF | POSITIF | POSITIF | **POSITIF** |
| 148 | 41 SA | POSITIF | POSITIF | POSITIF | POSITIF | **POSITIF** |
| 150 | 38 SA | NEGATIF | NEGATIF | NEGATIF | NEGATIF | **NEGATIF** |
| 151 | 16 SA | NEGATIF | NEGATIF | NEGATIF | NEGATIF | **NEGATIF** |
| 152 | 16 SA | NEGATIF | NEGATIF | NEGATIF | NEGATIF | **NEGATIF** |
| 154 | 36 SA | NEGATIF | NEGATIF | NEGATIF | NEGATIF | **NEGATIF** |
| 155 | 40 SA | NEGATIF | NEGATIF | NEGATIF | NEGATIF | **NEGATIF** |
| 156 | 34 SA | NEGATIF | NEGATIF | NEGATIF | NEGATIF | **NEGATIF** |
| 157 | 38 SA | NEGATIF | NEGATIF | NEGATIF | NEGATIF | **NEGATIF** |
| 158 | 36 SA | NEGATIF | NEGATIF | NEGATIF | NEGATIF | **NEGATIF** |
| 159 | 29 SA | NEGATIF | NEGATIF | NEGATIF | NEGATIF | **NEGATIF** |
| 161 | 18 SA | NEGATIF | NEGATIF | NEGATIF | NEGATIF | **NEGATIF** |
| 163 | 32 SA | POSITIF | NEGATIF | NEGATIF | NEGATIF | **NEGATIF** |
| 164 | 39SA+6j | POSITIF | NEGATIF | NEGATIF | NEGATIF | **POSITIF** |
| 165 | 40 SA | POSITIF | NEGATIF | NEGATIF | NEGATIF | **POSITIF** |
| 166 | 41 SA+3j | NEGATIF | NEGATIF | NEGATIF | NEGATIF | **NEGATIF** |
| 167 | 32 SA | NEGATIF | NEGATIF | NEGATIF | NEGATIF | **NEGATIF** |
| 168 | 16 SA | NEGATIF | NEGATIF | NEGATIF | NEGATIF | **NEGATIF** |
| 169 | 28 SA | NEGATIF | NEGATIF | NEGATIF | NEGATIF | **NEGATIF** |
| 170 | 40 SA | NEGATIF | NEGATIF | NEGATIF | NEGATIF | **NEGATIF** |
| 171 | 33 SA | NEGATIF | NEGATIF | NEGATIF | NEGATIF | **NEGATIF** |
| 172 | 20 SA | NEGATIF | NEGATIF | NEGATIF | NEGATIF | **NEGATIF** |
| 173 | 39 SA | NEGATIF | NEGATIF | NEGATIF | NEGATIF | **NEGATIF** |
| 174 | 41 SA | NEGATIF | NEGATIF | NEGATIF | NEGATIF | **NEGATIF** |
| 175 | 41 SA | POSITIF | POSITIF | POSITIF | POSITIF | **POSITIF** |
| 176 | 40 SA | POSITIF | POSITIF | POSITIF | POSITIF | **POSITIF** |
| 178 | 38 SA | NEGATIF | NEGATIF | NEGATIF | NEGATIF | **NEGATIF** |
| 180 | 24 SA | NEGATIF | NEGATIF | NEGATIF | NEGATIF | **NEGATIF** |
| 181 | 41 SA | NEGATIF | NEGATIF | NEGATIF | NEGATIF | **NEGATIF** |
| 182 | 40 SA | POSITIF | POSITIF | POSITIF | POSITIF | **POSITIF** |
| 183 | 39 SA | POSITIF | POSITIF | POSITIF | POSITIF | **POSITIF** |
| 184 | 41 SA | POSITIF | POSITIF | NEGATIF | POSITIF | **POSITIF** |
| 185 | 31 SA | NEGATIF | NEGATIF | NEGATIF | NEGATIF | **NEGATIF** |
| 186 | 34 SA | NEGATIF | NEGATIF | NEGATIF | NEGATIF | **NEGATIF** |
| 187 | 33 SA | NEGATIF | NEGATIF | NEGATIF | NEGATIF | **NEGATIF** |
| GM101 | 40 SA | NEGATIF | NEGATIF | NEGATIF | NEGATIF | **NEGATIF** |
| GM102 | 41 SA | POSITIF | NEGATIF | NEGATIF | NEGATIF | **POSITIF** |
| GM103 | 38 SA | NEGATIF | NEGATIF | NEGATIF | NEGATIF | **NEGATIF** |
| GM104 | 37 SA | NEGATIF | NEGATIF | NEGATIF | NEGATIF | **NEGATIF** |
| GM106 | 38 SA | NEGATIF | NEGATIF | NEGATIF | NEGATIF | **NEGATIF** |
| GM107 | 38 SA | NEGATIF | NEGATIF | NEGATIF | NEGATIF | **NEGATIF** |
| GM108 | 23 SA | NEGATIF | NEGATIF | NEGATIF | NEGATIF | **NEGATIF** |
| GM109 | 32 SA | NEGATIF | NEGATIF | NEGATIF | NEGATIF | **NEGATIF** |
| GM111 | 34 SA | NEGATIF | NEGATIF | NEGATIF | NEGATIF | **NEGATIF** |
| GM114 | 41 SA | NEGATIF | NEGATIF | NEGATIF | NEGATIF | **NEGATIF** |
| GM115 | 40 SA | NEGATIF | NEGATIF | NEGATIF | NEGATIF | **NEGATIF** |
| GM116 | 40 SA | NEGATIF | NEGATIF | NEGATIF | NEGATIF | **POSITIF** |
| GM117 | 37 SA | POSITIF | POSITIF | POSITIF | POSITIF | **POSITIF** |
| GM120 | 28 SA | NEGATIF | NEGATIF | NEGATIF | NEGATIF | **NEGATIF** |
| GM121 | 39 SA | POSITIF | POSITIF | POSITIF | POSITIF | **POSITIF** |
| GM122 | 18 SA | POSITIF | POSITIF | POSITIF | POSITIF | **POSITIF** |

| | | | | | | |
|---|---|---|---|---|---|---|
| Note SA = semaine d'aménorrhée | | | | | | |

L'interprétation du clinicien a été donnée après la fin de la grossesse en ayant connaissance de l'ensemble du dossier ce qui inclut les données cliniques et échographiques au moment où le test a été réalisé mais aussi les événements survenus par la suite jusqu'à la naissance. Le diagnostic de rupture n'était pas retenu lorsqu'aucun écoulement n'était visible au spéculum en dépit d'un test à la toux, que la quantité de liquide restait normale à l'échographie et que la patiente regagnait son domicile sans nouvel épisode d'écoulement pendant au moins 48 heures ou qu'une poche des eaux était nettement perçue sans écoulement clinique pour les patientes entrant en travail dans un délai de 48 heures. Un avis de rupture a été donné lorsqu'un écoulement avait été signalé et que l'examen au spéculum avec test de la toux objectivait un écoulement et/ou lorsque la poche des eaux n'était pas perçue y compris au cours du travail d'accouchement dans les 48 heures suivant le test. Les patientes sans écoulement objectivable avec un oligoamnios à l'échographie ont fait l'objet d'une surveillance rapprochée (toutes les 48 heures) ou d'un déclenchement sous 48 heures. Le diagnostic de rupture était retenu si l'écoulement récidivait formellement sous 48 heures ou si la poche des eaux n'était pas perçue y compris au cours du travail d'accouchement pour les patientes déclenchées.

La véracité du test a pu être vérifiée par un examen gynécologique complet permettant de confirmer ou d'infirmer le résultat.

21,6% des échantillons (22 cas sur 102) présentent une discordance entre l'un des trois marqueurs et les données cliniques qui représentent l'avis de l'obstétricien.

Le tableau 2 ci-dessous décrit des exemples de résultats des donnés cliniques et le tableau 3 les données cliniques et l'interprétation par le clinicien.

**Tableau 2 : tableau d'exemples de résultats avec les données cliniques pour des cas non concordants**

| **N° échantillon** | **Terme** | **Procédé de l'état de la technique** | **Dispositif de l'invention** | | ***INTERPRETATION*** | **Données Cliniques** |
|---|---|---|---|---|---|---|
| | | ***INTERPRETATION*** | ***IGFBP-1*** | ***AFP*** | | |
| 119 | 40 SA+2j | POSITIF | NEGATIF | NEGATIF | NEGATIF | NEGATIF |
| 163 | 32 SA | POSITIF | NEGATIF | NEGATIF | NEGATIF | NEGATIF |
| 112 | 40SA+ 4j | POSITIF | POSITIF | NEGATIF | POSITIF | POSITIF |
| 126 | 39SA+ 2J | POSITIF | POSITIF | NEGATIF | POSITIF | POSITIF |
| 136 | 41SA+ 4j | POSITIF | POSITIF | NEGATIF | POSITIF | POSITIF |
| 137 | 37SA+ 6j | POSITIF | POSITIF | NEGATIF | POSITIF | POSITIF |
| 184 | 41 SA | POSITIF | POSITIF | NEGATIF | POSITIF | POSITIF |
| Nombre faux positifs | | 2 | 0 | | | |
| Nombre faux négatifs | | 3 | 5 | | | |

| | | | | | | |
|---|---|---|---|---|---|---|
| Note SA = semaine d'aménorrhée J = jours | | | | | | |

**Tableau 3 : tableau comprenant des cas non concordants et l'analyse clinique**

| **N°échantillon** | **avis obstétricien** | **Terme (semaines d'aménorrhées)** | **COMMENTAIRES** |
|---|---|---|---|
| 119 | NEGATIF | 40+2j | 40 semaines d'aménorrhées +2 jours. Travail spontané Col ouvert à 4 cm avec poche des eaux perçues, pertes depuis 3 jours. |
| 163 | NEGATIF | 32 | 32 semaines d'aménorrhées. Suspicion de pertes au décours de vomissements post HGPO (hyperglycémie provoquée par voie orale). A l'échographie, liquide amniotique normal et patiente ressort d'hospitalisation. Pas d'écoulement pendant deux semaines. |
| 112 | POSITIF | 40+4j | 40 semaines d'aménorrhées + 4 jours écoulement visible. pH ne vire pas. |
| 126 | POSITIF | 39+2j | 39 semaines d'aménorrhées + 2 jours, signal pertes, pH ne vire pas. |
| 136 | POSITIF | 41+4j | 41 semaines d'aménorrhées +4 jours. La patiente signale des pertes. |
| 137 | POSITIF | 37 | 37 semaines d'aménorrhées +6 jours. La patiente signale des pertes. |
| 115 | POSITIF | 34+4j | Ecoulement franc à 34 semaines d'aménorrhées + 4jours avec diagnostic d'oligoamnios à l'échographie. Patiente hospitalisée en dépit d'un test ACTIM^{®} (marque déposée) PROM négatif tant la clinique est en faveur d'une rupture de membrane. Poursuite des pertes et accouchement spontané 4 jours après. |
| 184 | POSITIF | 40SA+5j | Rupture franche, écoulement visible. |

| | | | |
|---|---|---|---|
| Note SA = semaine d'aménorrhée J = jours | | | |

Le tableau 4 ci-dessous résume les résultats obtenus avec le procédé de l'invention par rapport au procédé de l'état de la technique.

**Tableau 4 :**

| | Procédé de l'état de la technique | Procédé de l'invention (IGFBP-1 + AFP) |
|---|---|---|
| Spécificité | 97,10% (67/69) | 100% (64/64) |
| Intervalle de confiance | 89,92 - 99,65 | 94,4 - 100 |
| Concordance avec la clinique | 95,10% | 94,60% |

Ainsi, le procédé de l'invention comprenant la détection de l'IGFBP-1 et de l'AFP permet d'obtenir une spécificité de 100%, permettant ainsi d'éliminer tous résultats faux positifs et de conclure lorsque l'AFP et l'IGFBP-1 sont détectés à la rupture des membranes foetales au contraire des dispositifs de l'art antérieur. Il s'agit là d'une amélioration majeure car les résultats faussement positifs des tests de l'état de l'art représentent un vrai problème pour le clinicien et la patiente car ils peuvent engendrer des actes médicaux inutiles (prise d'antibiotiques, induction du travail ou déclenchement d'une césarienne).

En outre, selon l'invention, les échantillons pour lesquels les résultats obtenus étaient AFP+/IGFBP1- (considéré comme non déterminé par le clinicien), une étape complémentaire de détection de l'IGFBP-1 avec un seuil de détection de 5 ng/mL a été réalisée. Ainsi il a été possible d'augmenter la sensibilité de la détection de la rupture des membranes foetales tel qu'indiqué dans le tableau 5 suivants :

**Tableau 5 :**

| | Procédé de l'état de la technique | Procédé de l'invention (IGFBP-1 + AFP) comprenant une étape supplémentaire de détection de l'IGFBP-1 |
|---|---|---|
| Spécificité | 97,10% (67/69) | 100% (69/649) |
| Intervalle de confiance | 89,92 - 99,65 | 94,79-100 |
| Nbre de non déterminés | 0 | 0 |
| Concordance avec la clinique | 95,10% | 95,10% |

Selon un autre mode de réalisation de l'invention, la cinétique de l'AFP a été prise en compte. En particulier, la concentration dans le liquide amniotique baisse en fin de grossesse. Ainsi, la détection ou non de l'AFP a été corrélée avec la date de prélèvement de l'échantillon. En particulier, l'échantillon a été classé en fonction de s'il a été prélevé avant ou pendant/ après la 39^{ème} semaine d'aménorrhée.

Le tableau 6 ci-dessous résume les résultats obtenus et la date de prélèvement

**Tableau 6**

| | Faux négatifs en AFP | Faux positifs en AFP |
|---|---|---|
| Population dont le terme est inférieure à 39SA | 0 | 5 |
| Population dont le terme est supérieur ou égale 39SA | 5 | 0 |

Comme démontré précédemment, dans ce mode de réalisation la rupture est détectée avec le procédé de l'invention avec certitude.

En outre, selon ce mode de réalisation, la population testée pour laquelle le test était AFP/IGFBP1 a été divisée en deux en fonction que l'échantillon provenait de patientes de terme inférieur à 39 semaines (sous groupe 1) ou de patientes de terme supérieur ou égal à 39 semaines (sous groupe 2). Dans le présent exemple, le sous groupe 1 correspond à 45 patientes parmi lesquelles aucun faux négatif n'a été mis en évidence et le sous groupe 2 correspond à 24 patientes chez lesquelles 5 faux négatifs sont détectés. La population testée pour laquelle le test était AFP+/IGFBP1-a été divisée en fonction que l'échantillon provenait de patientes de terme inférieur à 39 semaines (sous groupe 3) ou de patientes de terme supérieur ou égal à 39 semaines (sous groupe 4).

Tel que démontré dans le tableau 7 ci-dessous, le procédé de l'invention permet en excluant les patientes correspondant aux sous-groupes 2 et 3, de détecter avec une sensibilité et une spécificité de 100%, la rupture ou la non rupture des membranes foetales. A contrario, les procédés de l'état de la technique ne permettent pas d'obtenir de tels résultats. En particulier, des faux positifs et des faux négatifs sont obtenus avec les dispositifs connus sans possibilité d'identifier des sous-groupes de patientes chez lesquelles le résultat est fiable à 100%.

**Tableau 7**

| | Procédé de l'état de la technique | Procédé l'invention (IGFBP-1 + AFP) |
|---|---|---|
| Performances | 3 faux négatifs, 2 faux positifs | 0 faux positif, 0 faux négatif |
| Sensibilité | 90,91% (30/33) | 100% (24/24) |
| Intervalle de confiance | 75,67 - 98,08 | 85,75 - 100 |
| Spécificité | 97,10% (67/69) | 100% (48/48) |
| Intervalle de confiance | 89,92 - 99,65 | 92,60 - 100 |
| Nbre de cas non déterminés | 0 | 30/102 (29,4%) |
| Concordance avec la clinique | 95,10% | 100,00% |

Tel que démontré dans cet exemple le procédé de l'invention permet d'obtenir un résultat fiable de la rupture ou de la non rupture des membranes foetales permettant avantageusement de s'affranchir de tests et ou examens cliniques complémentaires.

### Listes des références

1. Rutanen et al. Radioimmunoassay of placenta protein 12 : levels in amniotic fluid, cord blood and serum of healthy adults, pregnant women and patients with trophoblastic disease. Am. J. Obstet. Gynecol. 1982
2. Rochelson et al. Rapid assay: possible application in the diagnosis of prématuré rupture of the membranes. Obstet Gynecol 1983; 62:414-418
3. Young-Han Kim et al. Vaginal fluid b-human chorionic gonadotropin level in the diagnosis of prématuré rupture of membranes. Acta Obstet Gynecol Scand 2005: 84: 802-805
4. Koninckx PR et al. Prolactin concentration in vaginal fluid: a new method for diagnosing ruptured membranes. Br J Obstet Gynaecol 1981;88:607-610
5. Rutanen et al, "Monoclonal antibodies to the 27-34K insulin-like growth factor binding protein" Biochem Biophys Res Commun 1988 ; 152 : 208
6. Pekonen et al, "A monoclonal antibody-based immunoradiometric assay for low molecular weight insulin-like growth factor binding protein/placental protein 12", J immunoassay 1989 ; 10 : 325-337
7. Beer et al. Qualification of cellulose nitrate membranes for lateral-flow assays, IVD technology, janvier 2002.
8. Anne Harwood Peruski et al. Methods for Détection and Identification of Infectious Disease and Biological Warfare Agents. Clinical and diagnostic laboratory immunology, July 2003, p. 506-513
9. Rutanen et al. Measurement of Insulin-like growth factor binding protein-1 in cervical/vaginal secrétions : comparison with the ROM-Check Membrane Immunoassay in the diagnosis of ruptured fetal membranes. Clinica Chimica Acta, 214 (1993), 73-81
10. Rutanen et al. Decidual transformation of human extrauterine mesenchymal cells is associated with is associated with the appearance of insulin like growth factor binding protein 1. J. Clin. Endocrinol. Metab. 1992 ; 72 : 27-31
11. Rutanen et al. Evaluation of a rapid strip test for insulin-like growth factor binding protein 1 in the diagnosis of foetal ruptured membranes. Clinica Chimica Acta. 253 ; (1996) 91-101
12. Kishida et al. Diagnosis of premature rupture of the membranes in peterm patients, using an improved AFP kit : comparison with ROM-Check and/or nitrazine test. European Journal of Obstetrics and Gynecology and Reproductive Biology 69 (1996) 77-82
13. Bell C. Secretory endometrial and decidual proteins : studies and clinical significance of a maternally derived group of pregnancy associated serum proteins. Human Reproduction. Vol. 1-3 129-143. 1986.
14. Nazimova et al. Blood Serum Content of PAMG-1 Protein Binding Insulin like Growth Factor 1 (Somatomedin C) in Patients with Diabetes Mellitus. Bullet. Experim. Biol. and Med. 166/9 September 1993

## Revendications

1. Procédé de détection *in-vitro* d'une rupture de membranes foetales comprenant une étape de recherche simultanée, dans un prélèvement de sécrétions vaginales ou cervicales, de l'alpha foeto protéine (AFP) et de la protéine 1 de liaison au facteur de croissance Insulinomimétique (IGFBP-1), procédé dans lequel une rupture de membrane foetale est détectée lorsque l'IGFBP-1 seule ou en combinaison avec l'AFP est détectée, l'absence de rupture est détectée lorsque ni l'IGFBP-1 ni l'AFP n'est détecté, et lorsque seule l'AFP est détectée, le procédé comprend éventuellement en outre une étape de détection de l'IGFBP-1 seule.

2. Procédé selon la revendication 1 dans lequel la recherche est effectuée avec au moins un anticorps de capture dirigé contre l'AFP et avec au moins un anticorps de capture dirigé contre l'IGFBP-1.

3. Procédé selon la revendication 1 ou 2 dans lequel la recherche est effectuée à partir d'un prélèvement de sécrétions vaginales ou cervicales dilué dans une solution tampon.

4. Procédé selon la revendication 3 dans lequel le procédé comprend l'ajustement d'au moins un des paramètres choisis parmi le volume, le pH, la force ionique du milieu tampon, ledit ajustement étant commun à l'AFP et à l'IGFBP-1.

5. Procédé selon la revendications 3 ou 4 dans lequel le pH de la solution tampon est de 5 à 10.

6. Procédé selon l'une quelconque des revendications 2 à 5, dans lequel les anticorps de capture anti AFP et anti IGFBP-1 sont sous forme sèche.

7. Procédé selon l'une quelconque des revendications 1 à 6 dans lequel ladite étape de recherche comprend une étape de révélation effectuée avec au moins un anticorps marqué anti AFP et au moins un anticorps marqué anti IGFBP1.

8. Procédé selon l'une quelconque des revendications 2 à 7 dans lequel le procédé comprend l'ajustement d'au moins un des paramètres choisis parmi la concentration des anticorps de capture et/ou la concentration des anticorps marqués, ledit ajustement étant spécifique à l'AFP ou à l'IGFBP-1.

9. Procédé selon la revendication 8, dans lequel ledit IGFBP-1 est détecté à partir d'une concentration de 1 à 10 ng/ml.

10. Utilisation du dispositif comprenant :
a. une zone (1) de dépôt d'un échantillon
b. une zone (2) comprenant des anticorps anti IGFBP-1 et des anticorps anti AFP marqués.
c. une zone (3) de révélation comprenant des anticorps de capture dirigé contre l'AFP et des anticorps de capture dirigé contre l'IGFPB-1 pour la mise en oeuvre du procédé selon l'une quelconque des revendications 1 à 9.

11. Utilisation du dispositif selon la revendication 10 dans lequel la zone de dépôt (1) est soit un papier absorbant soit un support en fibre de verre.

12. Utilisation du dispositif selon la revendication 10 ou 11 dans lequel la zone de révélation (3) est une membrane de nitrocellulose.

13. Utilisation du dispositif selon l'une quelconque des revendications 10 à 12 dans lequel la zone (2) est un support en fibre de verre.

## Patentansprüche

1. Verfahren zum In-vitro-Nachweis eines Blasensprungs, umfassend einen Schritt des gleichzeitigen Suchens von alpha-Fetoprotein (AFP) und dem insulinähnlichen Wachstumsfaktor-Bindungsprotein-1 (IGFBP-1) in einer Probe von Vaginal- oder Zervikalsekreten, Verfahren in dem der Blasensprung nachgewiesen ist, wenn IGFBP-1 allein oder in Verbindung mit AFP nachgewiesen ist, wobei das Ausbleiben eines Blasensprungs nachgewiesen ist, wenn weder IGFBP-1 noch AFP nachgewiesen ist und wenn nur AFB nachgewiesen ist, das Verfahren gegebenenfalls ferner einen Schritt zum Nachweis von IGFBP-1 allein umfasst.

2. Verfahren nach Anspruch 1, wobei die Suche mit mindestens einem gegen AFP gerichteten Fänger-Antikörper und mindestens einem gegen IGFBP-1 gerichteten Fänger-Antikörper ausgeführt wird.

3. Verfahren nach Anspruch 1 oder 2, wobei die Suche in einer Probe von mit einer Pufferlösung verdünnten Vaginalsekreten oder Zervikalsekreten ausgeführt wird.

4. Verfahren nach Anspruch 3, wobei das Verfahren das Einstellen mindestens eines, aus dem Volumen, dem pH-Wert, der Ionenstärke des Puffermediums ausgewählten Parameters umfasst, wobei das Einstellen für AFP und IGFBP-1 gemeinsam ist.

5. Verfahren nach Anspruch 3 oder 4 mit einem pH-Wert der Pufferlösung von 5 bis 10.

6. Verfahren nach einem der Ansprüche 2 bis 5, wobei die Anti-AFP- Fänger-Antikörper und die Anti-IGFBP-1-Fänger-Antikörper in trockener Form vorliegen.

7. Verfahren nach einem der Ansprüche 1 bis 6, wobei der Suchschritt einen Nachweisschritt umfasst, der mit mindestens einem anti-AFP markierten Antikörper und mindestens einem anti-IGFBP1 markierten Antikörper durchgeführt wird.

8. Verfahren nach einem der Ansprüche 2 bis 7, wobei das Verfahren das Einstellen mindestens eines aus der Konzentration der Antikörper-Fänger und/oder aus der Konzentration der markierten Antikörper ausgewählten Parameters umfasst, wobei das Einstellen jeweils für AFP oder IGFBP-1 spezifisch ist.

9. Verfahren nach Anspruch 8, wobei das IGFBP-1 ab einer Konzentration von 1 bis 10 ng/ml nachgewiesen wird.

10. Verwendung der Vorrichtung umfassend:
a. einen Bereich (1) zur Aufnahme der Probe
b. einen Bereich (2), der die markierten Anti-AFP und Anti-IGFBP-1 Antikörper umfasst
c. einen Bereich (3) zum Nachweis, der die gegen AFP gerichteten Antikörper-Fänger und die gegen IGFBP-1 gerichteten Antikörper-Fänger umfasst
zur Durchführung des Verfahrens nach einem der Ansprüche 1 bis 9.

11. Verwendung der Vorrichtung nach Anspruch 10, wobei der Aufnahmebereich (1) entweder ein absorbierendes Papier oder ein Glasfaser-Träger ist.

12. Vorrichtung nach Anspruch 10 oder 11, wobei der Nachweisbereich (3) eine Nitrocellulosemembran ist.

13. Verwendung der Vorrichtung nach einem der Ansprüche 10 bis 12, wobei der Bereich (2) ein Glasfaserträger ist.

## Claims

1. An *in-vitro* method of detection of a rupture of fetal membranes comprising a step of simultaneously searching, in a specimen of vaginal or cervical secretions, for alpha-fetoprotein (AFP) and for insulin-like growth factor-binding protein 1 (IGFBP-1), method wherein a rupture of fetal membranes is detected when IGFBP-1 is detected alone or in combination with AFP, the absence of rupture is detected when neither IGFBP-1 nor AFP is detected; and when AFP is only detected the method comprises eventually a further step of detection of IGFBP-1 only.

2. The method according to claim 1, wherein the search is carried out with at least one capture antibody directed against AFP and with at least one capture antibody directed against IGFBP-1.

3. The method according to claim 1 or 2, wherein the search is carried out using a specimen of vaginal or cervical secretions which is diluted in a buffer solution.

4. The method according to claim 3, wherein the method comprises the adjustment of at least one of the parameters chosen from the volume, the pH and the ionic strength of the buffer medium, said adjustment being common to AFP and to IGFBP-1.

5. The method according to claims 3 or 4, wherein the pH of the buffer solution is from 5 to 10.

6. The method as claimed in any one of claims 2 to 5, wherein the anti-AFP and anti-IGFBP-1 capture antibodies are in dry form.

7. The method as claimed in any one of claims 1 to 6, wherein said searching step comprises a visualizing step carried out with at least one anti-AFP labeled antibody and at least one anti-IGFBP-1 labeled antibody.

8. The method as claimed in any one of claims 1 to 7, wherein the process comprises the adjustment of at least one of the parameters selected from the concentration of the capture antibodies and/or the concentration of the labeled antibodies, said adjustment being specific to AFP or to IGFBP-1.

9. The method as claimed in claim 9, in which said IGFBP-1 is detected at a starting concentration from 1 to 10 ng/ml.

10. Use of a device comprising:
- . a zone (1) for depositing a sample,
- . a zone (2) comprising labeled anti-IGFBP-1 antibodies and labeled anti-AFP antibodies,
- . a visualizing zone (3) comprising capture antibodies directed against AFP and capture antibodies directed against IGFBP-1
for implementing the method as according in any one of claims 1 to 9.

11. Use of a device according to claim 10, wherein the depositing zone (1) is either an absorbent paper or a glass fiber support.

12. Use of a device as claimed in claim 10 or 11, wherein the visualizing zone (3) is a nitrocellulose membrane.

13. Use of a device as claimed in any one of claims 10 to 12, wherein the zone (2) is a glass fiber support.
